(19) Europäisches Patentamt
European Patent Office
Office européen des brevets

(11) **EP 3 472 335 B1**

(12) **EUROPEAN PATENT SPECIFICATION**

(45) Date of publication and mention
of the grant of the patent:
**25.12.2024 Bulletin 2024/52**

(21) Application number: **17732068.6**

(22) Date of filing: **16.06.2017**

(51) International Patent Classification (IPC):
*C12P 7/64* (2022.01)          *C12P 7/6458* (2022.01)
*C12P 7/649* (2022.01)

(52) Cooperative Patent Classification (CPC):
**C12P 7/64; C12P 7/6458; C12P 7/649;** Y02E 50/10

(86) International application number:
**PCT/EP2017/064851**

(87) International publication number:
**WO 2017/216382 (21.12.2017 Gazette 2017/51)**

(54) **REDUCTION OF PHOSPHOLIPIDS IN PHOSPHOLIPID-CONTAINING OIL MATERIAL**

REDUKTION VON PHOSPHOLIPIDEN IN PHOSPHOLIPIDHALTIGEM ÖLMATERIAL

RÉDUCTION DE PHOSPHOLIPIDES DANS UN MATÉRIAU D'HUILE CONTENANT DES PHOSPHOLIPIDES

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **16.06.2016 EP 16174870**

(43) Date of publication of application:
**24.04.2019 Bulletin 2019/17**

(73) Proprietor: **Novozymes A/S**
**2880 Bagsvaerd (DK)**

(72) Inventor: **NIELSEN, Per Munk**
**2880 Bagsvaerd (DK)**

(74) Representative: **NZ EPO Representatives**
**Krogshoejvej 36**
**2880 Bagsvaerd (DK)**

(56) References cited:
**EP-A2- 1 893 764     EP-B1- 1 893 764**

• **YANG LI ET AL: "Combined phospholipase and lipase catalysis for biodiesel production from phospholipids-containing oil",** BIOTECHNOLOGY AND BIOPROCESS ENGINEERING, vol. 20, 2015, pages 965 - 970, XP035563407

• **SILVIA CESARINI ET AL: "Moving towards a competitive fully enzymatic biodiesel process",** SUSTAINABILITY, vol. 7, 2015, pages 7884 - 7903, XP002762332, DOI: 10.3390/su7067884

• **SILVIA CESARINI ET AL: "Combining phospholipases and a liquid lipase for one-step biodiesel production using crude oils",** BIOTECHNOLOGY FOR BIOFUELS, vol. 7, 2014, pages 1 - 12, XP021180012

• **XIAOFEI JIANG ET AL: "Application of phospholipase A1 and phospholipase C in the degumming process of different kinds of crude oils",** PROCESS BIOCHEMISTRY, vol. 50, 2015, pages 432 - 437, XP002762337, DOI: 10.1016/ j.procbio.2014.12.011

• **TAO WEI ET AL: "Characterization of a novel thermophilic phospholipase B from Thermotoga lettingae TMO: applicability in enzymatic degumming of vegetable oils",** JOURNAL OF INDUSTRIAL MICROBIOLOGY AND BIOTECHNOLOGY, vol. 42, 13 January 2015 (2015-01-13), pages 515 - 522, XP035470736

• **AIXIA LIU ET AL: "Streptomyces violaceoruber phospholipase A2: expression in Pichia pastoris, properties, and application in oil degumming",** APPLIED BIOCHEMISTRY AND BIOTECHNOLOGY, vol. 175, 2015, pages 3195 - 3206, XP035460880

- **SANGEETA KANAKRAJ ET AL: "A comprehensive review on degummed biodiesel", BIOFUELS, vol. 7, no. 5, 21 April 2016 (2016-04-21), pages 537 - 548, XP002772830, DOI: 10.1080/17597269.2016.1168021**
- **SANGKOM SUBPHASO ET AL: "Effect of parameters on degumming of cashew nut shell", JOURNAL OF SCIENCE AND TECHNOLOGY OF THE MAHASARAKHAM UNIVERSITY, vol. 32, 2013, THAILAND, pages 475 - 480, XP002772833**
- **PATEL ET AL: "Castor oil: Properties, uses, and optimization of processing parameters in commercial production", LIPID INSIGHTS, vol. 9, 7 September 2016 (2016-09-07), pages 1 - 12, XP002772831, DOI: 10.4137/LPI.S40233**

Remarks:
The complete document including Reference Table(s) and the Sequence Listing(s) can be downloaded from the EPO website

# EP 3 472 335 B1

**Description**

**FIELD OF THE INVENTION**

The present invention provides a method for reducing the content of phospholipids in a phospholipid-containing oil material by converting the phospholipids into fatty acid alkyl esters (FAAE) and free fatty acids (FFA). The method can be used in degumming or as an enzymatic pretreatment before transesterification and/or biodiesel production, in particular chemically catalyzed transesterification and/or biodiesel production. However, the method can also be used as basic oleochemical in further downstream processes of the oleochemical industry.

**BACKGROUND ART**

Biodiesel is commonly produced by the transesterification of vegetable oil or animal fat feedstock. Nowadays, biodiesel is mainly produced from edible oils such as those from soybean, rapeseed, sunflower, or palm, by chemically reacting lipids with an alcohol. In order to make the process more economical and sustainable, the use of low value or non-edible feedstocks is investigated as an alternative for biodiesel production. In this context, non-refined (crude) soybean oil is an alternative solution already investigated for enzymatic transesterification. However, crude, non-degummed oils contain impurities as phosphorous compounds (phospholipids), which have to be removed for efficient transesterification. Crude soybean oil generally has a phosphorus (gums) content of 800 to 1,200 ppm, equivalent to 2-3% of phospholipids, which can cause problems during storage due to precipitate formation and water accumulation. Moreover, according to the legal specifications, phosphorus concentration in final biodiesel must be reduced to less than 10 ppm (EN14214:2003; ASTM D6751:2012).

For biodiesel production, a high content of phospholipids in the raw feedstocks means a concomitant loss of yield in free fatty alkyl ester (FAAE) production, as the fatty acids enclosed into the phospholipid molecules are not accessible for transesterification. Further, it has been found (Freedman) that the yield of methyl esters can be reduced from 67% to 86%, due to a large amount of free fatty acids in crude oil. Thus, a low content of both phospholipids and free fatty acids are important for high yields of free fatty alkyl esters (FAAE).

Various physical and chemical methods are used to degum oil (as described by Bochisch, M. in Fats and Oils Handbook, AOCS Press, 1998, p. 428-433). A process for enzymatic degumming is described by Clausen, K in Eur. J. Lipid Sci. Techno.103 (2001), 333-340. The key steps are citric acid treatment, pH adjustment to approximately 5.0, enzyme addition and mixing using a high shear mixer. This is usually followed by water addition and centrifugation to separate the hydrophobic and hydrophilic phases. For biodiesel production, current industrial practice favors the homogeneous alkaline transesterification, normally catalyzed by Na-metoxide, K-metoxide, NaOH or KOH or enzymatically catalyzed transesterification using lipase. The chemically catalyzed transesterification has some disadvantages as it requires low content of FFA (less than 0.5%) and water to avoid soap formation and loss of oil yield. However, this problem can be solved by an acid-catalyzed pretreatment. The disadvantages of this method is that it requires complete removal of water, high methanol to oil ratio, has a low reaction time and is highly energy consuming. Enzymatically catalyzed transesterification (using lipase), does not require low water and FFA content, however with few exceptions chemically catalyzed transesterification is currently preferred in the industry.

The use of phospholipase for enzymatic degumming of an edible oil (Clausen et al. Dansk kemi 88 (2002), 24-27, US 5,264,367; JP-A-2153997; and EP 622446), to reduce the phosphorus content of water degummed oils has been disclosed. So has a method of converting phospholipids into lyso-phospholipids by reacting a phospholipid-containing oil material with water, ethanol (6-40wt%) and a phospholipase A (WO2016/064848). Further, a combined degumming/-transesterification process using phospholipase and lipase together in a methanol system (EP1893764) has been disclosed.

Combining phospholipase and liquid lipase in a process for production of biodiesel has also been proposed by Cesarini (Cesarini et al. Biotechnology for Biofuels, 2014, 7:29). In Cesarini's process lipase and phospholipase are added to the reaction mixture at the same and the methanol is then added by pumping in a linear gradient for 10 hours, reaching a level corresponding to 1.5 eqv. Furthermore, Cesarini *et al.* have previously reviewed different natural tools which were developed for the synthesis of FAMEs from unrefined oils in a completely enzymatic single-step process (Cesarini et al. Sustainability 7; 7884-7903 (2015)).

Cesarini's studies combining phospholipases and lipid catalysts for biodiesel production have been repeated by Li (Li et al., Biotechnology and Bioprocess Engineering 20; 965-970 (2015)). Li has shown that the ability of a phospholipase to reduce phosphorous content in a soybean oil is greatly reduced by addition of approximately 5% methanol or more. Li concluded that to effectively combine enzymatic degumming and enzymatic production of biodiesel, a two-step method is required. The process includes a first step for removing phospholipids by incubation of the oil for 2 hours in the absence of alcohol, followed by a second step in which lipase and alcohol is added.

As a low content of both phospholipids, free fatty acids (FFAs) and water are important for high yields of free fatty alkyl esters (FAAE) during chemically catalyzed transesterification, new methods that reduce the phospholipid content and at the same time keep the FFA and water content at a minimum are needed.

## SUMMARY OF THE INVENTION

The present invention provides a method for reducing the content of phospholipids in a phospholipid-containing oil material by converting the phospholipids into fatty acid alkyl esters (FAAE) and free fatty acids (FFA). The method comprises reacting a phospholipid-containing oil material with one or more phospholipases in a system comprising 1-2.5 wt% short chain alcohol having 1 to 5 carbon atoms and water. The method can be used in degumming or as an enzymatic pretreatment before transesterification and/or biodiesel production, such as chemically catalyzed transesterification and/or biodiesel production, but it can also be used as basic oleochemical in further downstream processes of the oleochemical industry, or for producing free fatty alkyl esters (FAAE) from phospholipids.

The invention also provides the use of a phospholipase to catalyse the formation of fatty acid alkyl esters/biodiesel in a reaction mixture comprising a phospholipid-containing oil material, a short chain alcohol having 1 to 5 carbon atoms, and water, wherein the amount of said short chain alcohol in the reaction mixture is in the range of 1-2.5 wt%.

## DESCRIPTION OF FIGURES

Figure 1: The distribution of C17:0 fatty acid and the methyl ester of C17:0 in three assays (PLA and 1.5% methanol). Determined using the GC-FID method.

Figure 2: Comparison of phospholipase activity at 0.5 wt%, 1 wt% and 2 wt% methanol.

Figure 3: Comparison of phospholipase activity at 1.5 wt% methanol with control; no methanol present.

## DEFINITIONS

Biodiesel: Fatty acid alkyl esters (FAAE) of short chain alcohols such as fatty acid methyl ester (FAME) or fatty acid ethyl ester (FAEE) are referred to as biodiesel, as they can be used as an additive in or a replacement of fossil diesel.

**Crude oils:** Crude oils are unrefined oils, meaning that they still contain phospholipids that need to be eliminated during the process to biodiesel. The term "crude oil" refers to (also called a non-degummed oil) a pressed or extracted oil or a mixture thereof from, e.g. vegetable sources, including but not limited to acai oil, almond oil, babassu oil, blackcurrant seed oil, borage seed oil, canola oil, cashew oil, castor oil, coconut oil, coriander oil, corn oil, cottonseed oil, crambe oil, flax seed oil, grape seed oil, hazelnut oil, hempseed oil, jatropha oil, jojoba oil, linseed oil, macadamia nut oil, mango kernel oil, meadowfoam oil, mustard oil, neat's foot oil, olive oil, palm oil, palm kernel oil, palm olein, peanut oil, pecan oil, pine nut oil, pistachio oil, poppy seed oil, rapeseed oil, rice bran oil, safflower oil, sasanqua oil, sesame oil, shea butter, soybean oil, sunflower seed oil, tall oil, tsubaki oil walnut oil, varieties of "natural" oils having altered fatty acid compositions via Genetically Modified Organisms (GMO) or traditional "breading" such as high oleic, low linolenic, or low saturated oils ( high oleic canola oil, low linolenic soybean oil or high stearic sunflower oils).

**Degumming:** Degumming refers to a process in which the phospholipid content of a phospholipid containing oil material is reduced. A typical enzymatic degumming process consist of a treatment step with acid, NaOH and enzyme followed by centrifugation to separate the hydrophobic and hydrophilic phases. After removal of non-hydratable phospholipids, hydratable phospholipids, and lecithins (known collectively as "gums") from the oil to produce a degummed oil or fat product that can be used for food production and/or non-food applications, e.g. biodiesel. In certain embodiments, the degummed oil has the phospholipids content of less than 200 ppm phosphorous, such as less than 150 ppm phosphorous, less than 100 ppm phosphorous, less than (or less than about) 50 ppm phosphorous, less than (or less than about) 40 ppm phosphorous, less than (or less than about) 30 ppm phosphorous, less than (or less than about) 20 ppm phosphorous, less than (or less than about) 15 ppm phosphorous, less than (or less than about) 10 ppm phosphorous, less than (or less than about) 7 ppm phosphorous, less than (or less than about) 5 ppm phosphorous, less than (or less than about) 3 ppm phosphorous or less than (or less than about) 1 ppm phosphorous

**Fatty acid alkyl esters (FAAE):** Fatty acid alkyl esters are esters with a long carbon chain and an alkyl group, derived by transesterification fats with an alcohol. If the alcohol is methanol, the alkyl group in the fatty acid alkyl ester will be methyl, if the alcohol is ethanol, the alkyl group will be ethyl and so on.

**Fatty acid feedstock:** The term "fatty acid feedstock" is defined herein as a substrate comprising triglyceride. In addition to triglyceride, the substrate may comprise diglyceride, monoglyceride, free fatty acid or any combination thereof. Any oils and fats of vegetable or animal origin comprising fatty acids may be used as substrate for producing fatty acid alkyl esters in the process of the invention.

The fatty acid feedstock may be oil selected from the group consisting of: algae oil, castor oil, coconut oil (copra oil), corn oil, cottonseed oil, flax oil, fish oil, grape seed oil, hemp oil, jatropha oil, jojoba oil, mustard oil, canola oil, palm oil, palm stearin, palm olein, palm kernel oil, peanut oil, rapeseed oil, rice bran oil, safflower oil, soybean oil, sunflower oil, tall oil, and oil from halophytes, or any combination thereof.

The fatty acid feedstock may be fat selected from the group consisting of: animal fat, including tallow from pigs, beef and sheep, lard, chicken fat, fish oil, or any combination thereof.

The fatty acid feedstock may be crude, refined, bleached, deodorized, degummed, or any combination thereof.

Food quality oils and fats are expensive and therefore waste and by-products from their processing as well as non-food grade oils and fats have become increasingly attractive feedstock for fatty acid alkyl ester. Soap stock is the fraction of oil obtained in an oil refinery by treating the oil with a base to convert free fatty acids to soaps (e.g., sodium soaps). The soap stock usually contains a fraction of glycerides beside the soaps. Acid oil is the by-product from the oil refinery produced by acidification of soap stock to solubilize the soaps. It mainly contains free fatty acids (FFA) and acylglycerols. Distillates like Palm Fatty Acid Distillate (PFAD) is the by-product from oil refining coming from a distillation process used to eliminate free fatty acid from the oil.

The feedstock may be an intermediate product, a waste product or a by-product of oil or fat refining selected from the group consisting of: soap stock; acid oil; fatty acid distillates such as PFAD, soy fatty acid distillate, rapeseed fatty acid distillate, rice bran fatty acid distillate, poultry fat fatty acid distillate, beef tallow fatty acid distillate, etc.; gums from degumming; by-products from the production of omega-3 fatty acids derivates from fish oil; fat trap grease; yellow grease, and brown grease, free fatty acids like oleic acid; or fractions of oil obtained by physical separations; or any combinations thereof.

**Fatty acid methyl esters (FAEE):** Fatty acid ethyl esters are esters with a long carbon chain and a ethyl group, derived by transesterification of fats with ethanol.

**Fatty acid methyl esters (FAME):** Fatty acid methyl esters are esters with a long carbon chain and a methyl group, derived by transesterification of fats with methanol.

**Free fatty acids (FFA):** A free fatty acid is a carboxylic acid with a long carbon chain. Most naturally occurring fatty acids have an unbranched chain of an even number of carbon atoms, from 4 to 28. Free fatty acids are usually derived from fats (triglycerides (TAG), diglycerides (DAG), monoglyceride(DAG)), phospholipids or lyso-phospholipids.

- **Triglycerides** are formed by combining glycerol with three fatty acid molecules. The hydroxyl (HO-) group of glycerol and the carboxyl (-COOH) group of the fatty acid join to form an ester. The glycerol molecule has three hydroxyl (HO-) groups. Each fatty acid has a carboxyl group (-COOH).
- **Diglycerides** are formed by combining glycerol with two fatty acid molecules.
- **Monoglycerides** are formed by combining glycerol with one fatty acid molecule.

Lipase: The term lipase is defined herein as a polypeptide with a lipolytic activity which hydrolyses the carboxylic ester bond in glyceryl tributyrate, olein, pNP-butyrate and pNP-palmitate (triacylglycerol lipase, EC 3.1.1.3).

**Mature polypeptide:** The term "mature polypeptide" means a polypeptide in its final form following translation and any post-translational modifications, such as N-terminal processing, C-terminal truncation, glycosylation, phosphorylation, etc. In one aspect, the mature polypeptide may be predicted, such as by using the software SignalP (Nielsen et al., 1997, Protein Engineering 10: 1-6)]

**Phospholipases:** A phospholipase is referred to as an enzyme that hydrolyzes phospholipids into fatty acids and other lipophilic substances. There are four major classes, termed A, B, C and D, distinguished by the type of reaction which they catalyze:

- Phospholipase A - cleaves either the SN-1 acyl chain or the SN-2 acyl chain
- Phospholipase B - cleaves both SN-1 and SN-2 acyl chains
- Phospholipase C - cleaves before the phosphate, releasing diacylglycerol and a phosphate-containing head group.
- Phospholipase D - cleaves after the phosphate, releasing phosphatidic acid and an alcohol.

**Phospholipase A and B:** The term "phospholipase A" comprises a polypeptide having phospholipase A1 and/or phospholipase A2 activity (A1 or A2, EC 3.1.1.32 or EC 3.1.1.4), *i.e.,* hydrolytic activity towards one or both carboxylic ester bonds in phospholipids such as lecithin. A phospholipase having both A1 and A2 activity is also referred to as a phospholipase B. In addition to having phospholipase A activity, the polypeptides referred to as phospholipase A may also have lipase activity.

**Phospholipase C activity:** Phospholipase C (E.C. 3.1.4.11) removes the phosphate moiety in phospholipids such as lecithin to produce 1,2 diacylglycerol and phosphate ester.

**PI-Specific Phospholipase C/Phospholipase C with Pi-specificity:** The term "PI-specific phospholipase C" or "Phospholipase C with PI-specificity" relates to a polypeptide having activity towards phosphatidyl inositol (PI), meaning that its activity towards phosphatidylcholine (PC), phosphatidylethanolamine (PE), phosphatidic acid (PA) is low compared to the PI activity. PI-specific phospholipase C enzymes can either belong to the family of hydrolases and phosphodiesterases classified as EC 3.1.4.11 or to the family of lyases classified as EC 4.6.1.13. PI-specific phospholipase C activity may be determined according to the procedure described in Example 5. Preferably a PI-specific phospholipase C removes at least 30% PI from an oil or fat with at least 50 ppm PI when using the P-NMR assay of Example 5 at the optimal pH of the enzyme and an enzyme dosage of 10 mg/kg. More preferably it removes 40%, 50%, 60%, 70% or 80%, even more preferred it removes 90% and most preferred it removes between 90% and 100% of the PI in the oil or fat.

Preferably a PI-specific Phospholipase C removes at least 20% more PI when compared to the amount of PC, PE or PA it can remove, more preferred at least 30%, 40%, even more preferred at least 50% and most preferred at least 60% more PI when compared to the amount of PC, PE or PA it can remove.

**Phospholipase D activity:** Phospholipase D (E.C. 3.1.4.4) acts on phospholipids such as lecithin and produces 1,2-diacylglycerophosphate and base group.

**Phospholipid containing oil material:** Phospholipid containing oil material refers to any oil or fat substrate comprising phospholipids.

**Phospholipids:** Phospholipids are a class of lipids that generally consists of two hydrophobic fatty acid "tails" and a hydrophilic phosphate "head", joined together by a glycerol molecule.

**Short chain alcohol:** The alcohol used in the invention is a short-chain alcohol having 1 to 5 carbon atoms ($C_1$, $C_2$, $C_3$, $C_4$, or $C_5$). Preferred alcohols are methanol and ethanol.

**Sequence identity:** The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

**Transesterification:** Transesterification refers to a process in which the organic group R" of an ester is exchanged with the organic group R' of an alcohol.

**Variant:** The term "variant" means a polypeptide having phospholipase C activity comprising an alteration, i.e., a substitution, insertion, and/or deletion, at one or more (e.g., several) positions. A substitution means replacement of the amino acid occupying a position with a different amino acid; a deletion means removal of the amino acid occupying a position; and an insertion means adding an amino acid adjacent to and immediately following the amino acid occupying a position.

**DETAILED DESCRIPTION OF THE INVENTION**

The present invention relates to a method for converting phospholipids in a phospholipid-containing oil material into free fatty acids and fatty acid alkyl esters. The present inventors have surprisingly found that when performing phospholipase-catalyzed reduction of phospholipids in the presence limited amounts 1-2.5 wt% of short chain alcohol having 1 to 5 carbon atoms, the phospholipase is also able to catalyze a transesterification reaction so that production of fatty acid alkyl esters takes place concomitantly with the phosphorous reduction. Moreover, the present inventors have observed that the presence of such amounts of alcohol also results in more efficient phosphorous reduction. Such observations have not been made previously. In particular, the afore-mentioned studies by Cesarini *et al.* and Li *et al.* which investigated phospholipase-catalyzed reduction of phospholipids and lipase-catalysed production of fatty acid alkyl esters did not suggest that fatty acid alkyl esters could be produced in a phospholipase-catalyzed reaction.

An aspect of the invention is a phospholipid reducing process comprising: i) providing a reaction mixture comprising a phospholipid-containing oil material, one or more phospholipid-degrading enzymes, a short chain alcohol having 1 to 5 carbon atoms, and water, and ii) incubating of the reaction mixture; wherein, when incubating said reaction mixture, the amount of said short chain alcohol in the reaction mixture is in the range of 1-2.5 wt%, such as in the range of 1.2-2.5 wt%, 1-2.25 wt%, 1-2 wt%, 1.0-1.8 wt%, 1.2-2.0 or 1.2-1.8 wt%, and further wherein the reaction mixture is incubated in step ii) to provide a reacted oil material. The phospholipid-containing oil material can be any material comprising phospholipids and triglyceride, diglyceride, monoglyceride, free fatty acid or any combination thereof. In principle, any oils and fats of vegetable or animal origin comprising fatty acids and phospholipids may be used as substrate. The phospholipid-containing oil material could be, or could be derived from algae oil, canola oil, coconut oil, castor oil, copra oil, corn oil, distiller's corn oils, corn oil free fatty acid distillate, cottonseed oil, flax oil, fish oil, grape seed oil, hemp oil, jatropha oil, jojoba oil, mustard oil, canola oil, palm oil, palm stearin, palm olein, palm kernel oil, peanut oil, rapeseed oil, rice bran oil, safflower oil, soybean oil, sunflower oil, tall oil, oil from halophytes, and/or animal fat, including tallow from pigs, beef and sheep, lard, chicken fat, fish oil, palm oil free fatty acid distillate, soy oil free fatty acid distillate, soap stock fatty acid material, yellow grease, and brown grease or any combination thereof. The phospholipid-containing oil material may be crude, refined, bleached, deodorized, degummed, or any combination thereof. The phospholipid-containing oil material may be an intermediate product, a waste product or a by-product of oil or fat refining selected from the group consisting of: soap stock; acid oil; fatty acid distillates such as PFAD, soy fatty acid distillate, rapeseed fatty acid distillate, rice bran fatty acid distillate, poultry fat fatty acid distillate, beef tallow fatty acid distillate, etc.; gums from degumming; by-products from the production of omega-3 fatty acids derivates from fish oil; fat trap grease; yellow grease, and brown grease, free fatty acids like oleic acid; or fractions of oil obtained by physical separations; or any combinations thereof.

In one embodiment said reaction mixture in step ii) is incubated under conditions allowing said one or more phospholipid-degrading enzymes to catalyze hydrolysis and/or transesterification of phospholipids in said phospholipid-containing oil material.

In one embodiment, the amount of said short chain alcohol having 1 to 5 carbon atoms is added to the reaction mixture within a period of 1 hour prior to or at the onset of incubation of the reaction mixture. The alcohol should be added such that the amount of said short chain alcohol is between 1-2.5 wt% when the enzyme is added to the reaction mixture.

The short chain alcohol used should preferably be an alcohol with 1-3 carbon atoms. In one embodiment, said short chain alcohol is selected from a group consisting of methanol, ethanol, propanol and combinations thereof.

In one embodiment, said short chain alcohol is ethanol. In one embodiment, said short chain alcohol is methanol. The type of short chain alcohol used, determines which type of FAEE that will be a product of the reaction.

It is desirable to have a low amount of water in the reaction mixture. In one embodiment, the water content of the reaction mixture during incubation is in the range of 0,3-5wt% or 0,3-4wt% or 0,3-3,5wt% or 0,3-2,8wt% or 0,3-2,5wt% or 0,3-2wt% or 1-2wt% or 1,5-2wt% or 2-2,8wt% or 2-3,5wt% or 1,5-2,8wt% or 2-3wt% or 1,5-3,5wt% or 1,5-4wt% or 2-4wt% or 2-5wt%.

The skilled person can easily realize that a longer reaction time will allow the reaction of more phospholipids with the phospholipid-degrading enzymes than a shorter reaction time. However, since this method has various industrial applications, the incubation time depends on the industrial use. In one embodiment, the reaction mixture is incubated for a period of 20-1400 minutes, such as 120-1400 minutes, 240-1400 minutes, 20-1000 minutes, 60-1000 minutes, 60-800 minutes, 60-500 minutes, 20-500 minutes or 20-300 minutes or 20-180 minutes or 20-150 minutes or 20-120 minutes. It is the "gist" of the present invention is to incubate the reaction mixture formed according to the invention, containing oil, a short chain alcohol having 1 to 5 carbon atoms, water and phospholipase for a period of time thus specified, the amount of said short chain alcohol in the reaction mixture is in the range of 1-2.5 wt%, before additional enzyme with lipase activity to further catalyze the formation of fatty acid alkyl esters.

In one embodiment, 50wt% or more or 55wt% or more or 60wt% or more or 65wt% or more or 70wt% or more or 75wt% or more or 80wt% or more or 90% or more or 95% or more of the phospholipids in the phospholipid-containing oil material are reacted by said phospholipid-degrading enzymes in the reaction mixture. "Reacted" means that at least one reaction occurs between a phospholipid-degrading enzyme and a phospholipid, changing the structure of the reacted phospholipids.

The present inventors found that when reacting a phospholipid-containing oil material with one or more phospholipases, water and a short chain alcohol having 1 to 5 carbon atoms, the amount of said short chain alcohol in the reaction mixture is in the range of 1-2.5 wt%, the phospholipids are converted to free fatty acids (FFA) or free fatty alkyl esters (FAAE). Without wishing to be bound by theory, the present inventors believe that a low water content and addition of a short chain alcohol having 1 to 5 carbon atoms (1-2.5 wt%) to the reaction mixture facilitate the formation of FAAE from phospholipids, as the short chain alcohol can take the place as a nucleophile instead of water and thus convert some of the phospholipids into FAAE instead of FFA.

In one embodiment, the reaction mixture is incubated under conditions such that the phospholipids of the oil material are converted to free fatty alkyl esters (FAAE) by 20% or more, such as 25% or more or 30% or more or 35% or more or 40% or more or 50% or more or 60% or more or 65% or more or 70% or more or 75% or more or 80% or more or 85% or more or 90% or more. The type of free fatty alkyl esters formed depends on the short chain alcohol added to the reaction mixture. The short chain alcohol having 1 to 5 carbon atoms can be selected from a group consisting of: methanol, ethanol, propanol or combinations thereof. If the short chain alcohol is methanol, the reaction mixture is incubated under conditions such that the phospholipids of the oil material is converted to free fatty methyl esters (FAME) by 20% or more or 25% or more or 30% or more or 35% or more or 40% or more or 50% or more or 60% or more or 65% or more or 70% or more or 75% or more or 80% or more or 85% or more or 90% or more. If the short chain alcohol is ethanol, the reaction mixture is incubated under conditions such that the phospholipids of the oil material is converted to FAEE by 20% or more or 25% or more or 30% or more or 35% or more or 40% or more or 50% or more or 60% or more or 65% or more or 70% or more or 75% or more or 80% or more or 85% or more or 90% or more. If the short chain alcohol is propanol, the reaction mixture is incubated under conditions such that the phospholipids of the oil material is converted to FAPE by 20% or more or 25% or more or 30% or more or 35% or more or 40% or more or 50% or more or 60% or more or 65% or more or 70% or more or 75% or more or 80% or more or 85% or more or 90% or more.

In one embodiment, phospholipids in the phospholipid-containing oil material are converted to FFA and FAAE (FFA:FAAE) in a ratio which is less than or equal to 80:20 and more than or equal to 10:90. If the short chain alcohol is methanol, phospholipids in the phospholipid-containing oil material are converted to FFA and FAME (FFA:FAME) in a ratio which is less than or equal to 80:20 and more than or equal to 10:90. If the short chain alcohol is ethanol, phospholipids in the phospholipid-containing oil material are converted to FFA and FAEE (FFA:FAEE) in a ratio which is less than or equal to 80:20 and more than or equal to 10:90. If the short chain alcohol is propanol, phospholipids in the phospholipid-containing oil material are converted to FFA and FAEE (FFA:FAPE) in a ratio which is less than or equal to 80:20 and more than or equal to 10:90.

The one or more phospholipid-degrading enzyme may be a polypeptide having phospholipase activity, acyltransferase activity or having phospholipase activity, as well as having acyltransferase activity, e.g., a polypeptide selected from the polypeptides disclosed in WO 2003/100044, WO 2004/064537, WO 2005/066347, WO 2008/019069, WO 2009/002480, and WO 2009/081094. Acyltransferase activity may be e.g., determined by the assays described in WO 2004/064537. In one embodiment, one or more phospholipid-degrading enzymes comprise one or more phospholipases.

In one embodiment, said one or more phospholipid-degrading enzymes can be selected from a group consisting of: phospholipase A1 from *Thermomyces lanuginosus* and a phospholipase C having activity towards phosphatidylinositol.

The phospholipase may be selected from the polypeptides disclosed in WO 2008/036863 and WO 20003/2758. Suitable phospholipase preparations are PURIFINE(R) (available from Verenium) and LECITASE(R) ULTRA (available from Novozymes A/S). An enzyme having acyltransferase activity is available as the commercial enzyme preparation LYSOMAX(R) OIL (available from Danisco A/S).

In specific embodiments the phospholipid-degrading enzyme is selected from the group consisting of:

a) a polypeptide having at least 90% sequence identity to the mature polypeptide of SEQ ID NO: 1;
b) a variant of the mature polypeptide of SEQ ID NO: 1 comprising a substitution, deletion, and/or insertion at one or more positions; and
c) a fragment of the polypeptide of (a) or (b) that has phospholipase A activity.

In one embodiment, the variant in b) differs by up to 10 amino acids, e.g., 1, 2, 3, 4, 5, 6, 7, 8, 9, or 10, from the mature polypeptide of SEQ ID NO: 1.

In particular, the fragment in c) may have a length of 250-273 amino acid residues, such as 260-273 amino acid residues,

In some embodiments the phospholipid degrading enzyme comprises, consists essentially of or consists of the amino acid sequence set forth in SEQ ID NO: 1.

In specific embodiments, the phospholipase C having activity towards phosphatidylinositol is selected from the group consisting of:

a) a polypeptide having at least 90% sequence identity to the mature polypeptide of SEQ ID NO: 2;
b) a variant of the mature polypeptide of SEQ ID NO: 2 comprising a substitution, deletion, and/or insertion at one or more positions; and
c) a fragment of the polypeptide of (a) or (b) that has phosphatidylinositol phospholipase C activity.

In one embodiment, the variant in b) differs by up to 10 amino acids, e.g., 1, 2, 3, 4, 5, 6, 7, 8, 9, or 10, from the mature polypeptide of SEQ ID NO: 2.

In particular, the fragment in c) may have a length of 260-296 amino acid residues, such as 270-296 amino acid residues,

In one embodiment, said one or more phospholipid degrading enzymes comprise a phospholipase A1 from *Thermomyces lanuginosus* and a phospholipase C having activity towards phosphatidylinositol.

In specific embodiments, the phospholipid-degrading enzyme comprises a phospholipase A1 selected from the group consisting of:

a) a polypeptide having at least 90% sequence identity to the mature polypeptide of SEQ ID NO: 1 ;
b) a variant of the mature polypeptide of SEQ ID NO: 1, comprising a substitution, deletion, and/or insertion at one or more positions; and

c) a fragment of the polypeptide of (a) or (b) that has phospholipase A activity;

in combination with a phospholipase C selected from the group consisting of:

a) a polypeptide having at least 90% sequence identity to the mature polypeptide of SEQ ID NO: 2;
b) a variant of the mature polypeptide of SEQ ID NO: 2 comprising a substitution, deletion, and/or insertion at one or more positions; and
c) a fragment of the polypeptide of (d) or (e) that has phosphatidylinositol phospholipase C activity.

In a further embodiment, the process comprises adjusting the pH of the reaction mixture to be between 2.5 and 7.0, between preferably 2.5 and 6, preferably between 3.0 and 5.5, preferably between 3.5 and 5.0 and most preferred between 3.0 to 4.5.

The phospholipase A may be selected from Lecitase® Ultra and Quara® LowP, available from Novozymes, Bagsvaerd, Denmark

The dosage of phospholipid-degrading enzymes depends on the specific enzymes used, the content of the phospholipid containing oil material, the water content and the amount and type of said short chain alcohol used in the reaction mixture. It will be within the capacity of the skilled person to determine the optimum dosage of the enzyme. However, preferably said one or more phospholipid-degrading enzymes is/are dosed such that 50wt% or more or 55wt% or more or 60wt% or more or 65wt% or more or 70wt% or more or 75wt% or more or 80wt% or more of the phospholipids in the phospholipid-containing oil material are reacted by said phospholipid-degrading enzymes in the reaction mixture.

In one embodiment, the dosage of phospholipase A1 is in the range of 0.05-1.0 mg enzyme protein (EP) /kg phospholipid containing oil, such as in the range of 0.1-1.0 mg enzyme protein/kg phospholipid containing oil.

In one embodiment, the dosage of phospholipase A1 in combination with a phospholipase C with PI specificity is in the range of 0.2-1.5 mg enzyme protein/kg phospholipid-containing oil.

The one or more phospholipid-degrading enzyme may be a polypeptide having phospholipase activity only, or a polypeptide having phospholipase activity as well as having some lipase activity.

In one embodiment, said one or more phospholipid-degrading enzymes contain little or no lipase activity. In the context of the invention, it is preferred that the lipase activity it is not the main activity of the polypeptide. However, a polypeptide with phospholipase or acyltransferase activity might also have some lipase activity. Preferably, the lipase activity of said one or more phospholipid-degrading enzymes in said reaction mixture is such that 5% or less or 4% or less or 3% or 2% or less or 1% or less or 0.5% or less of the triglycerides present in the reaction mixture are reacted during the incubation period.

The alcohol content should not exceed 2.5 wt% as higher alcohol levels may reduce the enzyme activity of the phospholipid degrading enzymes. As a consequence, in a preferred embodiment, a mixture of said phospholipid containing oil material and said short chain alcohol is admixed with the one or more phospholipid-degrading enzymes and water. The advantage of this, is that the alcohol content is already between 1-2,5wt% at the time the phospholipid-degrading enzyme and water is added to the mixture. However, the order of mixing will depend on the industrial use. In another embodiment, a mixture of said phospholipid containing oil material and said short chain alcohol having 1 to 5 carbon atoms is admixed with NaOH to adjust pH, before adding an enzyme composition comprising one or more phospholipid degrading enzymes and water. In yet another embodiment the process of mixing comprises the following steps: i) Incubation said phospholipid-containing oil material and said short chain alcohol having 1 to 5 carbon atoms with citric acid for a period between 30-90 minutes; ii) Adjusted pH; e.g. with NaOH; and then iii) Adding the one or more phospholipid degrading enzymes and water.

In one embodiment, the reaction mixture temperature during said incubation is from 30-70°C or 30-65°C or 30-60°C or 30-55°C or 35-70°C or 35-65°C or 35-60°C or 40-70°C or 40-65°C or 40-60°C or 40-55°C or 45-70°C or 45-65°C or 45-55°C or 45-58°C or 40-58°C or 40-59°C or 45-58°C or 48-58°C or 48-59°C.

In the process according to the invention, the reaction mixture is incubated in step ii) to provide a reacted oil material. The reacted oil material may comprise a hydrophobic phase comprising glycerides (mono-acylglycerides (MAG), di-acylglycerides (DAG), tri-acylglycerides (TAG), fatty alkyl esters (FAAE) and free fatty acids (FFA); and a hydrophilic phase comprising water, alcohol such as methanol, phosphor and phosphor-degrading enzymes.

In one embodiment, after the reaction mixture has been incubated, the reacted oil material is divided into at least two phases, comprising: A hydrophobic phase comprising phospholipids, lyso-phospholipids, glycerides (Monoglyceride (MAG), diglyceride (DAG), triglyceride (TAG)), free fatty alkyl esters (FAAE) and free fatty acids (FFA) and a hydrophilic phase comprising water, methanol, phosphor and phospholipid-degrading enzymes.

The fatty acids and alkyl esters normally lost during phase separation by centrifugation can be retained by omitting the separation step normally used in degumming, due to the small amounts of water and methanol present in the present process. Instead, the water in the reaction mixture can be reduced by evaporated and the remaining phosphor can be removed by downstream processing. In one embodiment, after the reaction mixture has been incubated, the water and methanol is separated from the reacted oil material by evaporation. In another embodiment, after the reaction mixture has been incubated, the hydrophobic oil phase is separated from the hydrophilic phase by centrifugation. In another embodiment, after the reaction mixture has been incubated, the hydrophobic phase is separated from the hydrophilic phase by water addition followed by centrifugation. This is a conventional degumming method, wherein the water addition facilitates hydration of some of the phospholipids and/or lyso-phospholipids left in the reacted oil material, which allows them to move to the hydrophilic phase and be separated from the oil phase during centrifugation.

In one embodiment, the present invention is used as a pretreatment before chemically catalyzed transesterification of said phospholipid-containing oil material. In another embodiment, said reacted oil material is used as a feedstock in a chemically catalyzed transesterfication process.

The reacted oil material may be used as a feedstock in an acid catalyzed transesterification process, In particular, the transesterification process may be catalyzed by a catalyst selected from the group consisting of $H_2SO_4$, HCl, $BF_3$, $H_3PO_4$ and sulfonic acids. In the process according to the invention, the reacted oil material may be used as a feedstock in an alkali catalyzed transesterification process, In particular, the transesterification process may be catalyzed by a catalyst selected from the group consisting of NaOH, CHsONa and KOH.

In one embodiment, the reacted oil material is used for biodiesel production. In one embodiment, the reacted oil material is used for biodiesel production using a non-enzymatic process for the transesterification. In one embodiment, the reacted oil material is used for biodiesel production using an enzymatic process for the transesterification.

In currently preferred embodiments according to the invention the reacted oil material is used as a feedstock in transesterification process or is used for biodiesel production, and there is no or essentially no separation of said hydrophobic phase and said hydrophilic phase before the transesterification process or the biodiesel production. In these embodiments, steps are not taken to remove phosphorous from other constituents of the reacted oil material, such as the FAAE, the glycerides and the FFA, before lipase is added and the transesterification/production of biodiesel is initiated. As the skilled person will realize, however, it may be desirable to reduce the amount of water in the reacted oil material, before adding lipase and proceeding with the transesterification or production of biodiesel.

In one embodiment, the reacted oil material is dried before the transesterification process, said reacted material having a water content less than 1.0w/w%, or less than 0.5w/w%, or less than 0.3w/w% after drying.

In another aspect, the invention provides the use of a phospholipase to catalyze the formation of fatty acid alkyl esters/biodiesel in a reaction mixture comprising a phospholipid-containing oil material, a short chain alcohol having 1 to 5 carbon atoms, and water, wherein the amount of said alcohol in the reaction mixture is in the range of 1-2.5 wt%, such as in the range of 1.2-2.5 wt%, 1-2.25 wt%,1-2 wt%, 1.0-1.8 wt%, 1.2-2.0 wt% or 1.2-1.8 wt%. It is to be understood that the features disclosed above in relation to the process, will also apply to this aspect of the invention.

The oil material obtainable by the present invention can be used in several contexts. Fatty acid alkyl esters are used in an extensive range of products and as synthetic intermediates. Some of their industrial applications include use as lubricants, plasticizers, antirust agents, drilling and cutting oils, and starting materials for synthesis of superamides and fatty alcohols. Certain embodiments of the present invention in particular relate to fuels. Fatty acid alkyl esters of short-chain alcohols are non-toxic, biodegradable and an excellent replacement wholly or partly for petroleum-based fuel due to the similarity in cetane number, energy content, viscosity and phase changes to those of petroleum-based fuels. A composition produced by the process of the present invention may consist of a mixture of at least two or even three of the following components: FAAE; triglyceride; diglyceride; monoglycerides; glycerol; and water. The composition may potentially be refined or purified by methods known in the art such as distillation (including flash evaporation, stripping, and deodorization); phase separation; extraction; and drying. The purpose of such refining could be to remove or recover one or more of the above-mentioned components from the composition. Examples include, but are not limited to, drying for the removal of water; phase separation for the removal of glycerol; and distillation for the isolation of FAAE. Hence, the crude reactant mixture (composition) can be applied without further refining, or refined by one or more methods. This may comprise separating the phase comprising the FAAE from the glycerol-water phase and further processing the phase with an immobilized lipase to increase the FAAE content.

**EXAMPLES**

**Analytical procedures:**

**Phospholipid Composition by LC-MS**

**Materials**

Chloroform (anhydrous ≥99%), dry methanol (Hydranal®, Æ0.01% water), ammonium acetate (HPLC grade, ≥99%) and acetonitrile (anhydrous ≥99.8%). The phospholipids used as calibration stocks and internal standard were PA (18:0/18:0), PC (18:0/18:0), PE (18:0/18:0), PI (18:0/18:0), PS (18:0/18:0), LPA (18:0), LPC (18:0), LPE (18:0), LPI (18:0).

All chemicals were purchased from Sigma Aldrich (St. Louis, MO, USA).

**Sample preparation and calibration stocks**

1500 µl of chloroform/methanol/IS (50:50:1 mg/l) was added directly to the Eppendorf tube containing a pre-weighted amount of oil (≥30 g) and mixed 10 minutes at 250 rpm (Eppendorf™ Thermomixer C). The content was subsequently transferred to a glass LC-vial and encapsulated. Standard solutions were obtained by dilution of a stock solution containing all phospholipids mentioned in materials dissolved in chloroform/methanol (50:50). This stock solution was also used as internal standard (IS).

**Instrumentation**

The system was set up with a column temperature of 40°C, an injection volume of 2 µl, flow rate of 0.4 ml/min and a total run time of 16 minutes. Mobil phase A was ACN/water/NH4Ac (50:50:5 mM) and phase B was ACN/water/NH4Ac (5:95:5 mM). Elution was isocratic 1% A until 2.75 minutes, followed by a linear gradient to 10% A at 6 minutes, 40% A at 9.50 minutes and 1% A at 10 minutes, followed by an isocratic run until 15.10 minutes. MS was set to scan from 400 to 900 m/z ions in both positive and negative mode and data processed using MassLynx/Excalibur (Waters).

**Quantification of FAME and FFA by GC-FID**

**Materials**

Dry methanol (Hydranal®, Æ0.01% water), formic acid (Merck, 98-100%), stearic acid (Grade I, 098.5%), palmitic acid (099%), Supelco 37 component FAME mix (CRM47885). All chemicals were purchased from Sigma Aldrich (St. Louis, MO, USA).

**Method**

To the 2 ml Eppendorf tube containing approximately 100 mg of oil (accurate mass determined previous) was added 1500 µl methanol/formic acid (10 wt%). The solution was afterwards mixed extensively, centrifuged and 500 µl transferred to 1.5 ml GC- vial. To that was added further 500 µl methanol/formic acid solution.

**Instrumentation**

Sample injection volume was set to 1 µl and oven program to 80°C (1 min), to 200°C at 20°C/min and a hold time of 1 min, then to 240 °C at 6°C/min, and finally a hold time of 5 min. A constant gas flow of 1.60 ml/min was used.

**Example 1:**

Investigation of phospholipase activity at methanol concentrations of 0.5, 1.0, 1.5 and 2.0 wt%

**Method**

*Enzymatic degumming with MeOH*

Sodium hydroxide (10 ppm, 0.05 M) and *MeOH* (0.5, 1.0, 1.5 and 2.0 wt%) were added to a 100 mL squared bottle containing 30.0 g of preheated crude soybean oil (40°C) and incubated for 20 minutes at 40°C and 250 rpm (New Brunswick™ Innova® 44). The given phospholipase formulation (Lecitase Ultra; available from Novozymes, Bagsvaerd, Denmark) was diluted with ultrapure water (18.4 MΩ·cm at 25 °C) to achieve a concentration corresponding to 0.517 ppm EP (weight of EP relative to weight of crude oil and a total water concentration of 3.5 wt% after addition. After the addition of phospholipase solution, the suspensions were shaken and sonicated for 5 min using a Branson Ultrasonic Cleaner at 40°C, and subsequently placed in the afore mentioned horizontal incubator at 40°C and 250 rpm.

*Enzymatic degumming with citric acid pretreatment*

The traditional enzymatic degumming process was conducted in accordance with the Cowan and Nielsen protocol, with an initial citric acid pretreatment of the crude soybean oil. Citric acid (650 ppm, 100 g/l) was added to a 100 mL squared bottle containing 30.0 g of preheated crude soybean oil (40°C), shaken and incubated for 45 minutes at 40°C and 250 rpm (New Brunswick™ Innova® 44). The content was subsequently neutralized by addition of sodium hydroxide (1 M, 1.5 equivalents to citric acid) and further incubated for 20 minutes at 40°C and 250 rpm. The phospholipase addition and the subsequent steps are identical to the method described in the section above.

*Sampling*

Sampling were conducted at approximately 0, 20, 40, 120 and 1440 minutes counting from last enzyme addition (≥3 minutes delay from first to last addition). Samples of approximately 270 and 700 $\mu$L were transferred from the emulsion to pre-weighted 2 ml Eppendorf tube, after thorough shaking of the container, and placed in a thermomixer (Eppendorf™ Thermomixer Comfort) at 99°C for exactly 6 minutes at 300 rpm. Subsequently, the total weight of the Eppendorf tubes were determined and stored at -18°C until analysis.

**Results**

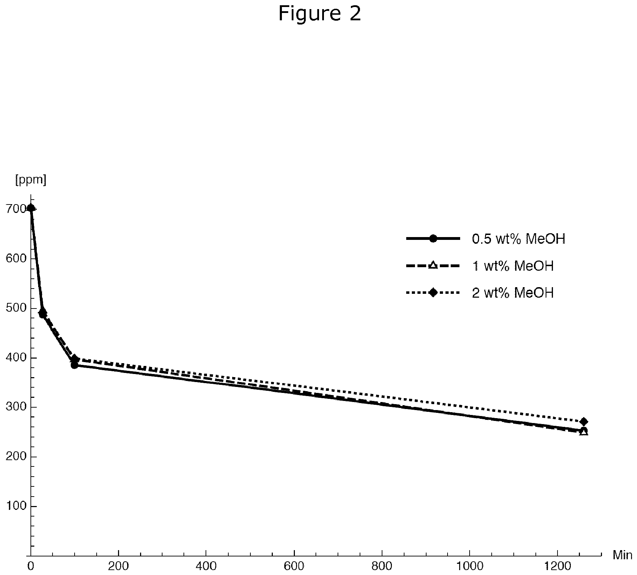

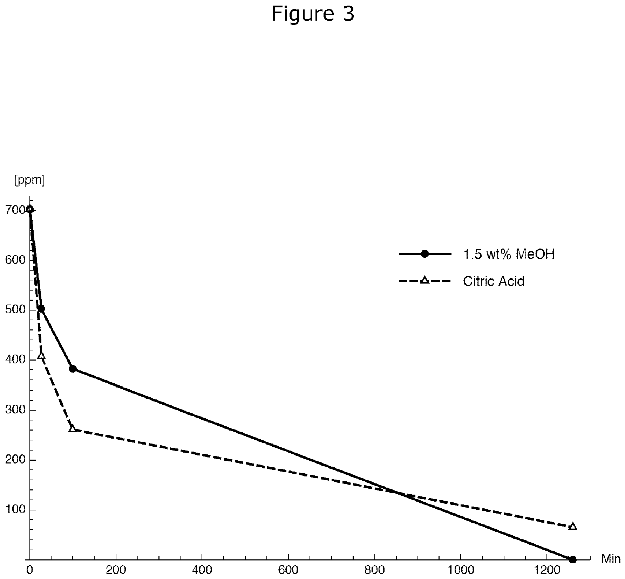

As evident from figure 2 approximately the same phospholipase activity is found initially in all assays. However, the differences become apparent at the end measurements, where the assays with 0.5, 1.0 and 2.0 wt% methanol seems to stall at the same phospholipid level. The assay having a methanol concentration of 1.5 wt% shows no intact phospholipids left at the P-NMR spectra, suggesting that this concentration is sufficient to facilitate the hydrolysis of phospholipids without a predominant inhibition of the phospholipase. Comparing this assay with the controls of citric acid pretreatment reveals a reduced initial rate of the methanol system, but a better long term hydrolysis of the phospholipids, cf. figure 3.

The remarkable resembling of the three assays of 0.5, 1.0 and 2.0 wt% methanol is though interesting, as the underlying mechanism are expected to be quite different. For the assays containing 1.0 wt% or less, a reduced phospholipid activity is expected due to the insufficient amounts of methanol to accommodate the physical change of the phospholipid system that facilitate hydrolysis, whereas the assay containing 2.0 wt% methanol a reduced phospholipase activity is expected due to a gradual inhibition. One could theorize, that the latter observation is in fact not an inhibition, but a physical change of the phospholipid system due to the appearance of the second interaction of biphasic effect.

**Example 2 (PLA assay with MeOH/EtOH)**

With this example, the facilitating effect of methanol and ethanol on the overall phospholipase activity in a degumming assay is established.

**Materials**

The soybean crude oil utilized in all assays was obtained from Loius Dreyfus Company, US, Batch No. BAS-2015-00014. The oil has been partitioned into 1 liter containers and stored at 5°C. PLA (SEQ ID NO: 1, 20 mg EP/mL), Sodium hydroxide solution (1.0 M), analytical grade methanol (≥99.7%), analytical grade ethanol, and citric acid (99%), were all purchased from Sigma Aldrich (St. Louis, MO, USA).

**Method**

*Enzymatic degumming with MeOH/EtOH*

Sodium hydroxide (10 ppm, 0.05 M) and *MeOH/EtOH* (1.5 wt%) were added to a 100 mL squared bottle containing 30.0 g of preheated crude soybean oil (40°C) and incubated for 20 minutes at 40°C and 250 rpm (New Brunswick™ Innova® 44). The given phospholipase formulation was diluted with ultrapure water (18.4 M$\Omega$·cm at 25 °C) to achieve a concentration corresponding to 0.517 ppm EP by the addition 3.50 wt% of the solution (including the added NaOH solution). By this procedure, an even distribution of enzyme in the emulsion, and a better frame of reference, were obtained together with a total water content of 3.5 wt%. After the addition of phospholipase solution, the suspensions were shaken and sonicated for 5 min using a Branson Ultrasonic Cleaner at 40°C, and subsequently placed in the aforementioned horizontal incubator at 40°C and 250 rpm.

*Enzymatic degumming with citric acid pretreatment*

The traditional enzymatic degumming process was conducted in accordance with the Cowan and Nielsen protocol, with an initial citric acid pretreatment of the crude soybean oil. Citric acid (650 ppm, 100 g/l) was added to a 100 mL squared bottle containing 30.0 g of preheated crude soybean oil (40°C), shaken and incubated for 45 minutes at 40°C and 250 rpm (New Brunswick™ Innova® 44). The content was subsequently neutralized by addition of sodium hydroxide (1 M, 1.5 equivalents to citric acid) and further incubated for 20 minutes at 40°C and 250 rpm. The phospholipase addition and the subsequent steps are identical to the method described in the section above.

*Sampling*

Sampling were conducted at approximately 0, 20, 40, 120 and 1440 minutes counting from last enzyme addition (≥3 minutes delay from first to last addition). Samples of approximately 270 and 700 $\mu$L were transferred from the emulsion to pre-weighted 2 ml Eppendorf tube, after thorough shaking of the container, and placed in a thermomixer (Eppendorf™ Thermomixer Comfort) at 99°C for exactly 6 minutes at 300 rpm. Subsequently, the total weight of the Eppendorf tubes was determined and stored at -18°C until analysis.

**Table 1:** LC-MS data on phospholipid (PL) and lipo-phospholipid (LPL) content (ppm phosphor) of the PLA assay at 24 hours. Mean and standard error are based on inter-day duplicates.

|  | LPL | PL | Total |
|---|---|---|---|
| **PLA, 650 ppm citric acid, 1.5 eqs. NaOH** | 84.6 $\pm$ 2.88 | 112.99 $\pm$ 4.25 | 197.59 $\pm$ 7.13 |
| **PLA, 1.5 wt% methanol** | 77.4 $\pm$ 8.66 | 3.99 $\pm$ 0.48 | 81.39 $\pm$ 9.13 |
| **PLA, 1.5 wt% ethanol** | 92.3 | 19.2 | 111.5 |

LC-MS determination of the 24-hour samples show a considerable reduction (LC-MS: 58.7$\pm$4.3%) in phospholipid content in the PLA assay of 1.5 wt% methanol compared to the standard degumming procedure of citric acid pretreated oil. An increased phospholipid reduction most likely facilitated by the partitioning of methanol, increasing fluidity and area per phospholipid, and hereby promoting the transfer of substrates to the active site.

The application of methanol in the enzymatic degumming of crude oils is of great potential, as a reduction of phospholipid content not only would increase product yield by the released FFA/FAME into the oil phase, the loss of neutral oil caused by aggregated phospholipids would be minimized.

**Example 3: PLA assay with MeOH and PLC**

With this example, the combined effect of phospholipase A and phospholipase C activity on phospholipid/lyso-phospholipid content in a degumming assay was investigated.

**Materials**

The soybean crude oil utilized in all assays was obtained from Louis Dreyfus Company, US, Batch No. BAS-2015-00014. The oil has been partitioned into 1 liter containers and stored at 5°C. PLA (SEQ ID NO: 1, 20 mg EP/mL), PI specific PLC (mature polypeptide of SEQ ID NO: 2 or 3), Sodium hydroxide solution (1.0 M), analytical grade methanol (≥99.7%), analytical grade ethanol, and citric acid (99%), were all purchased from Sigma Aldrich (St. Louis, MO, USA).

**Method**

*Enzymatic degumming with MeOH*

Sodium hydroxide (10 ppm, 0.05 M) and *MeOH* (1.5 wt%) were added to a 100 mL squared bottle containing 30.0 g of preheated crude soybean oil (40°C) and incubated for 20 minutes at 40°C and 250 rpm (New Brunswick™ Innova® 44). The given phospholipase formulation was diluted with ultrapure water (18.4 M$\Omega$·cm at 25 °C) to achieve a concentration corresponding to 0.517 ppm EP by the addition 3.50 wt% of the solution (including the added NaOH solution). By this procedure, an even distribution of enzyme in the emulsion, and a better frame of reference, were obtained together with a total water content of 3.5 wt%. After the addition of phospholipase solution, the suspensions were shaken and sonicated for 5 min using a Branson Ultrasonic Cleaner at 40°C, and subsequently placed in the aforementioned horizontal incubator at 40°C and 250 rpm.

*Enzymatic degumming with citric acid pretreatment*

The traditional enzymatic degumming process was conducted in accordance with the Cowan and Nielsen protocol, with an initial citric acid pretreatment of the crude soybean oil. Citric acid (650 ppm, 100 g/l) was added to a 100 mL squared bottle containing 30.0 g of preheated crude soybean oil (40°C), shaken and incubated for 45 minutes at 40°C and 250 rpm (New Brunswick™ Innova® 44). The content was subsequently neutralized by addition of sodium hydroxide (1 M, 1.5 equivalents to citric acid) and further incubated for 20 minutes at 40°C and 250 rpm. The phospholipase addition and the subsequent steps are identical to the method described in the section above.

*Sampling*

Sampling were conducted at approximately 0, 20, 40, 120 and 1440 minutes counting from last enzyme addition (≥3 minutes delay from first to last addition). Samples of approximately 270 and 700 $\mu$L were transferred from the emulsion to pre-weighted 2 ml Eppendorf tube, after thoroughly shaking of the container, and placed in a thermomixer (Eppendorf™ Thermomixer Comfort) at 99°C for exactly 6 minutes at 300 rpm. Subsequently, the total weight of the Eppendorf tubes was determined and stored at -18°C until analysis (LCMS).

**Table 2:** LC-MS data on phospholipid (PL) and lyso-phospholipid (LPL) content (ppm phosphor) at 24 hour of two combinations of PLA and PLC, and a reference assay with PLA only. All assays have a total EP of 0.517 ppm and 1.5 wt% methanol. Mean and standard error are based on inter-day duplicates.

| | PC | PE | PI | PA | Total PL |
|---|---|---|---|---|---|
| **PLA** | 1.59 ± 0.03 | 7.59 ± 5.73 | 0.56 ± 0.06 | 1.92 ± 0.13 | 11.70 ± 5.57 |
| **PLA + PLC (5:1)** | 1.58 ± 0.09 | 2.02 ± 0.00 | 0.14 ± 0.03 | 2.23 ± 0.04 | 5.97 ± 0.09 |
| **PLA + PLC (2:1)** | 1.71 ± 0.06 | 1.86 ± 0.28 | 0.21 ± 0.05 | 2.69 ± 0.45 | 6.47 ± 0.28 |
| | LPC | LPE | LPI | LPA | Total LPL |
| **PLA** | 7.84 ± 0.03 | 31.60 ± 1.25 | 49.90 ± 0.43 | 3.19 ± 0.02 | 92.50 ± 1.69 |
| **PLA + PLC (5:1)** | 10.80± 0.10 | 44.90 ± 0.55 | 4.26 ± 0.43 | 5.07 ± 0.08 | 65.00 ± 0.79 |
| **PLA + PLC (2:1)** | 11.10± 0,46 | 46.50 ± 1.41 | 2.06 ± 0.17 | 5.42 ± 0.28 | 65.10 ± 2.32 |

LC-MS determination of the 24-hour samples show a considerable reduction in phospholipid and lyso-phospholipid content in the PLA assay containing both PLA (SEQ ID NO: 1) and PLC (SEQ ID NO: 2 or 3) compared to samples only containing PLA, suggesting that a combination of a PLA and a PLC may provide a better degumming than a PLA alone.

**Example 4: PLA assay**

This example serves to confirm the production of FAME from phospholipids and the partitioning of methanol into the phospholipid monolayer of the reverse micelle, by quantification of the FAME/FFA ratio of the C17:0 fatty acid positioned on the synthetic substrate of phosphatidylcholine.

**Materials**

The soybean crude oil utilized in all assays was obtained from Louis Dreyfus Company, US, Batch No. BAS-2015-00014. The oil has been partitioned into 1 liter containers and stored at 5°C. PLA (SEQ ID NO: 1, 20 mg EP/mL), Sodium hydroxide solution (1.0 M), analytical grade methanol (≥99.7%), and 1,2-diheptadecanoyl-sn-glycero-3-phosphocholine (≥98%) were all purchased from Sigma Aldrich (St. Louis, MO, USA).

**Method**

1,2-diheptadecanoyl-sn-glycero-3-phosphocholine (C17PC, 50 $\mu$l) was transferred to three 2 ml Eppendorf tubes and left to evaporate overnight. Sodium hydroxide (87 $\mu$l, 0.05 M corresponding to 10 ppm) and methanol (332 $\mu$l corresponding to 1.5 wt%) was added to a 50 ml centrifuge tube containing 17.487 g of preheated crude soybean oil, and incubated for 20 minutes at 60°C and shaken regularly. To the pre-weighted Eppendorf tubes approximately 1 gram of pretreated crude oil was transferred, total weight determined, and added two 3 mm solid-glass beads. The Eppendorf tubes were subsequently placed for 2 hours in a thermomixer (EppendorfTM Thermomixer C) at 60°C and 900 rpm with 4 intervening 5 minutes sonications (Branson Ultrasonic Cleaner). The PLA formulation were diluted with ultrapure water (18.4 M ·cm at 25 °C) to achieve a concentration corresponding to 0.517 ppm EP by the addition 3.50 wt% of the solution (including the added NaOH solution). After cooling of the pretreated oil (to 40°C) and subsequent addition of the PLA solution, the suspensions were shaken and sonicated for 5 min at 40°C, and subsequently placed in the thermomixer at 40°C and 600 rpm. Samples for GC-FID of approximately 120 μL were transferred from the emulsion to pre-weighted 2 ml Eppendorf tube after 24 hours' incubation, using the procedure described in example 1.

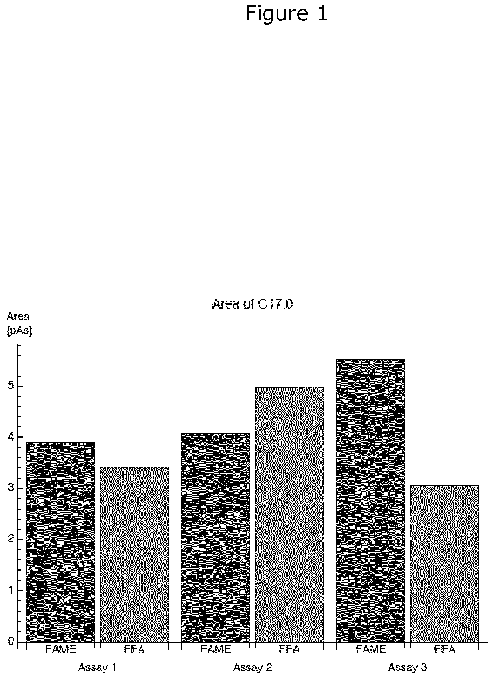

Data from this experiment is illustrated in figure 1. The data suggests that the phospholipids present in the crude oil are both subject to a hydrolysis and a transesterification/esterification in a ratio not reflecting that of the water/methanol concentration in the suspension. This gives an explicit confirmation, that a methanol partitioning into the phospholipid monolayer of the reverse micelle is taking place.

### Example 5: Methanol degumming

**Enzymes:** Lecitase Ultra (commercially available from Novozymes A/S, Bagsvaerd, Denmark);

Quara LowP: (commercially available from Novozymes A/S, Bagsvaerd, Denmark).

**Oil:** Standard soybean with approx. 600ppm P.

### Procedure

Water: 0.8 - 2.5%;
Methanol: 1.5%;
Enzyme dosage: 30 - 90ppm;
Reaction time: 2-6 hours.

Samples with PLA (SEQ ID NO: 5) are added 200ppm citric acid before enzyme addition. After incubation the samples are centrifuged and samples are taken for analysis (FFA, FAME, P). After that the oil phase is mixed with 5% water and placed in a rotator at room temperature for 30 min. The samples were centrifuged and aliquots were taken (for determination of free fatty acids (FFA), Fatty Methyl esters (FAME), phosphorous (P)).

### Sample set-up

|    | Enzyme | Enzyme dosage, mg EP/kg oil | Water, % | Reaction time, hrs |
|----|--------|------------------------------|----------|---------------------|
| 1  | Lecitase Ultra | 0.571 | 0.8 | 2 |
| 2  | Lecitase Ultra | 0.571 | 0.8 | 6 |
| 3  | Lecitase Ultra | 0.571 | 2.5 | 2 |
| 4  | Lecitase Ultra | 0.571 | 2.5 | 6 |
| 5  | Lecitase Ultra | 1.551 | 0.8 | 2 |
| 6  | Lecitase Ultra | 1.551 | 0.8 | 6 |
| 7  | Lecitase Ultra | 1.551 | 2.5 | 2 |
| 8  | Lecitase Ultra | 1.551 | 2.5 | 6 |
| 9  | PLA (SEQ ID NO: 5) | 0.142 | 0.8 | 2 |
| 10 | PLA (SEQ ID NO: 5) | 0.142 | 0.8 | 6 |
| 11 | PLA (SEQ ID NO: 5) | 0.142 | 2.5 | 2 |
| 12 | PLA (SEQ ID NO: 5) | 0.142 | 2.5 | 6 |

(continued)

| | Enzyme | Enzyme dosage, mg EP/kg oil | Water, % | Reaction time, hrs |
|---|---|---|---|---|
| 13 | PLA (SEQ ID NO: 5) | 0.427 | 0.8 | 2 |
| 14 | PLA (SEQ ID NO: 5) | 0.427 | 0.8 | 6 |
| 15 | PLA (SEQ ID NO: 5) | 0.427 | 2.5 | 2 |
| 16 | PLA (SEQ ID NO: 5) | 0.427 | 2.5 | 6 |

**Results:**

| | Enzyme | Enzyme dosage, mg EP/kg oil | Water, % | Reaction time, hrs. | FFA % | FFA % After water wash |
|---|---|---|---|---|---|---|
| 1 | Lecitase Ultra | 0.571 | 0.8 | 2 | 0.88 | 0.83 |
| 2 | Lecitase Ultra | 0.571 | 0.8 | 6 | 0.88 | 0.86 |
| 3 | Lecitase Ultra | 0.571 | 2.5 | 2 | 1.09 | 1.07 |
| 4 | Lecitase Ultra | 0.571 | 2.5 | 6 | 1.21 | 1.19 |
| 5 | Lecitase Ultra | 1.551 | 0.8 | 2 | 1.00 | 0.97 |
| 6 | Lecitase Ultra | 1.551 | 0.8 | 6 | 1.06 | 1.06 |
| 7 | Lecitase Ultra | 1.551 | 2.5 | 2 | 1.22 | 1.19 |
| 8 | Lecitase Ultra | 1.551 | 2.5 | 6 | 1.29 | 1.28 |
| 9 | PLA (SEQ ID NO: 5) | 0.142 | 0.8 | 2 | 0.85 | 0.80 |
| 10 | PLA (SEQ ID NO: 5) | 0.142 | 0.8 | 6 | 0.91 | 0.86 |
| 11 | PLA (SEQ ID NO: 5) | 0.142 | 2.5 | 2 | 0.90 | 0.90 |
| 12 | PLA (SEQ ID NO: 5) | 0.142 | 2.5 | 6 | 0.96 | 0.93 |
| 13 | PLA (SEQ ID NO: 5) | 0.427 | 0.8 | 2 | 0.89 | 0.86 |
| 14 | PLA (SEQ ID NO: 5) | 0.427 | 0.8 | 6 | 0.91 | 0.91 |
| 15 | PLA (SEQ ID NO: 5) | 0.427 | 2.5 | 2 | 0.93 | 0.91 |
| 16 | PLA (SEQ ID NO: 5) | 0.427 | 2.5 | 6 | 1.07 | 1.03 |

| | Enzyme | Enzyme dosage, mg EP/kg oil | Water, % | Reaction time, hrs. | FAME % | FAME % After water wash |
|---|---|---|---|---|---|---|
| 1 | Lecitase Ultra | 0.571 | 0.8 | 2 | 0.26 | 0.22 |
| 2 | Lecitase Ultra | 0.571 | 0.8 | 6 | 0.32 | 0.28 |
| 3 | Lecitase Ultra | 0.571 | 2.5 | 2 | 0.44 | 0.40 |
| 4 | Lecitase Ultra | 0.571 | 2.5 | 6 | 0.82 | 0.79 |
| 5 | Lecitase Ultra | 1.551 | 0.8 | 2 | 0.71 | 0.68 |
| 6 | Lecitase Ultra | 1.551 | 0.8 | 6 | 1.32 | 1.26 |
| 7 | Lecitase Ultra | 1.551 | 2.5 | 2 | 0.90 | 0.88 |
| 8 | Lecitase Ultra | 1.551 | 2.5 | 6 | 1.77 | 1.76 |
| 9 | PLA (SEQ ID NO: 5) | 0.142 | 0.8 | 2 | 0.27 | 0.24 |
| 10 | PLA (SEQ ID NO: 5) | 0.142 | 0.8 | 6 | 0.46 | 0.41 |

(continued)

|  | Enzyme | Enzyme dosage, mg EP/kg oil | Water, % | Reaction time, hrs. | FAME % | FAME % After water wash |
|---|---|---|---|---|---|---|
| 11 | PLA (SEQ ID NO: 5) | 0.142 | 2.5 | 2 | 0.23 | 0.20 |
| 12 | PLA (SEQ ID NO: 5) | 0.142 | 2.5 | 6 | 0.40 | 0.37 |
| 13 | PLA (SEQ ID NO: 5) | 0.427 | 0.8 | 2 | 0.50 | 0.45 |
| 14 | PLA (SEQ ID NO: 5) | 0.427 | 0.8 | 6 | 0.81 | 0.79 |
| 15 | PLA (SEQ ID NO: 5) | 0.427 | 2.5 | 2 | 0.31 | 0.29 |
| 16 | PLA (SEQ ID NO: 5) | 0.427 | 2.5 | 6 | 0.64 | 0.61 |

|  | Enzyme | Enzyme dosage, mg EP/kg oil | Water, % | Reaction time, hrs. | P, ppm | P, ppm; after water wash |
|---|---|---|---|---|---|---|
| 1 | Lecitase Ultra | 0.571 | 0.8 | 2 | 108 | 82 |
| 2 | Lecitase Ultra | 0.571 | 0.8 | 6 | 85 | 58 |
| 3 | Lecitase Ultra | 0.571 | 2.5 | 2 | 22 | 18 |
| 4 | Lecitase Ultra | 0.571 | 2.5 | 6 | 5.0 | 4.0 |
| 5 | Lecitase Ultra | 1.551 | 0.8 | 2 | 24 | 19 |
| 6 | Lecitase Ultra | 1.551 | 0.8 | 6 | 4.6 | 4.7 |
| 7 | Lecitase Ultra | 1.551 | 2.5 | 2 | 5.9 | 5.9 |
| 8 | Lecitase Ultra | 1.551 | 2.5 | 6 | 3.7 | 3.8 |
| 9 | PLA (SEQ ID NO: 5) | 0.142 | 0.8 | 2 | 32 | 17 |
| 10 | PLA (SEQ ID NO: 5) | 0.142 | 0.8 | 6 | 10 | 7.2 |
| 11 | PLA (SEQ ID NO: 5) | 0.142 | 2.5 | 2 | 26 | 26 |
| 12 | PLA (SEQ ID NO: 5) | 0.142 | 2.5 | 6 | 8.2 | 5.7 |
| 13 | PLA (SEQ ID NO: 5) | 0.427 | 0.8 | 2 | 13 | 8.8 |
| 14 | PLA (SEQ ID NO: 5) | 0.427 | 0.8 | 6 | 6.9 | 5.7 |
| 15 | PLA (SEQ ID NO: 5) | 0.427 | 2.5 | 2 | 10 | 15 |
| 16 | PLA (SEQ ID NO: 5) | 0.427 | 2.5 | 6 | 7.1 | 5.4 |

### Conclusions

The methanol degumming concept was investigated comparing two different phospholipases, Lecitase Ultra and a PLA having the amino acid sequence set forth in SEQ ID NO: 4. The enzymes were tested at dosages of 30 and 90 ppm, and at two different reaction times: 2 and 6 hours. Samples were analyzed after reaction/centrifugation as well as after washing the oil phase with 5% water followed by centrifugation. Reduction of phosphorous was achieved with both enzymes. FFA content was around 1% and FAME around 0.5%. As FFA in crude oil is 1.1%, there was a net reduction of FFA by esterification. Interestingly, there is no increase in FFA, as would be expected if phospholipase catalyzed hydrolysis of phospholipids was conducted in the absence of alcohol. Washing the oil phase with 5% water did not change the overall picture.

### Claims

1. A phospholipid reducing process comprising

   i) Providing a reaction mixture comprising a phospholipid-containing oil material, one or more phospholipid-degrading enzymes, a short chain alcohol having 1 to 5 carbon atoms, and water, and

ii) Incubating of the reaction mixture;

wherein, when incubating said reaction mixture, the amount of said short chain alcohol in the reaction mixture is in the range of 1-2.5 wt%,

and further wherein the reaction mixture is incubated in step ii) to provide a reacted oil material.

2. The process according to claim 1, wherein the reacted oil material comprises a hydrophobic phase comprising glycerides; and a hydrophilic phase comprising water, alcohol, phosphor and phosphor-degrading enzymes.

3. The process according to any of the preceding claims, wherein in step ii) the reaction mixture is incubated under conditions allowing said one or more phospholipid-degrading enzymes to catalyze hydrolysis and/or transesterfication of phospholipids in said oil material.

4. The process according to any of the preceding claims, wherein said amount of said short chain alcohol having 1 to 5 carbon atoms is added to the reaction mixture within a period of 1 hour prior to or at the onset of step ii).

5. The process according to any of the preceding claims, wherein said short chain alcohol is selected from a group consisting of methanol, ethanol, propanol and combinations thereof.

6. The process according to any of the preceding claims, wherein the water content of the reaction mixture during incubation is in the range of 0,3-5wt%.

7. The process according to any of the preceding claims, wherein in step ii) the reaction mixture is incubated under conditions such that the phospholipids of the oil material is converted to FAAE by 20% or more.

8. The process according to any of the preceding claims, wherein phospholipids in the phospholipid-containing oil material are converted to FFA and FAAE (FFA:FAAE) in a ratio which is less than or equal to 80:20 and more than or equal to 10:90.

9. The process according to any of the preceding claims, wherein one or more phospholipid-degrading enzymes comprise one or more phospholipases.

10. The process according to any of the preceding claims, wherein said one or more phospholipid degrading enzymes comprise a phospholipase A1 from *Thermomyces lanuginosus* and a phospholipase C having activity towards phosphatidylinositol.

11. The process according to any of the preceding claims, wherein the dosage of phospholipase A1 is in the range of 0.05-1.0 mg enzyme protein/kg phospholipid containing oil.

12. The process according to any of the preceding claims, wherein the amount of lipase activity in said reaction mixture is such that 5% or less of the triglycerides present in the reaction mixture are reacted during the incubation in step ii) of claim 1.

13. The process according to any of the preceding claims, wherein a mixture of said phospholipid containing oil material and said short chain alcohol is admixed with the one or more phospholipid-degrading enzymes and water.

14. The process according to any of the preceding claims, wherein said process is used as a pretreatment before chemically catalyzed transesterification of said phospholipid-containing oil material.

15. The process according to any of the preceding items, wherein said reacted oil material is used as a feedstock in transesterification process or is used for biodiesel production, and wherein there is no or essentially no separation of said hydrophobic phase and said hydrophilic phase before the transesterification process or the biodiesel production

16. Use of a phospholipase to catalyse the formation of fatty acid alkyl esters/biodiesel in a reaction mixture comprising a phospholipid-containing oil material, a short chain alcohol having 1 to 5 carbon atoms, and water, wherein the amount of said alcohol in the reaction mixture is in the range of 1-2.5 wt%.

**Patentansprüche**

1.  Phospholipid-Reduktionsverfahren, umfassend

    i) Bereitstellen eines Reaktionsgemisches, das ein phospholipidhaltiges Ölmaterial, ein oder mehrere phospholipidabbauende Enzyme, einen kurzkettigen Alkohol mit 1 bis 5 Kohlenstoffatomen und Wasser umfasst, und
    ii) Inkubieren des Reaktionsgemisches;
    wobei beim Inkubieren des Reaktionsgemisches die Menge des kurzkettigen Alkohols im Reaktionsgemisch im Bereich von 1-2,5 Gew.-% liegt,
    und wobei des Weiteren das Reaktionsgemisch in Schritt ii) inkubiert wird, um ein umgesetztes Ölmaterial bereitzustellen.

2.  Verfahren nach Anspruch 1, wobei das umgesetzte Ölmaterial eine hydrophobe Phase, die Glyceride umfasst, und eine hydrophile Phase, die Wasser, Alkohol, Phosphor und phosphorabbauende Enzyme umfasst, umfasst.

3.  Verfahren nach einem beliebigen der voranstehenden Ansprüche, wobei in Schritt ii) das Reaktionsgemisch unter Bedingungen inkubiert wird, die es dem einen oder den mehreren phospholipidabbauenden Enzymen ermöglichen, eine Hydrolyse und/oder Umesterung von Phospholipiden in dem Ölmaterial zu katalysieren.

4.  Verfahren nach einem beliebigen der voranstehenden Ansprüche, wobei die Menge des kurzkettigen Alkohols mit 1 bis 5 Kohlenstoffatomen innerhalb eines Zeitraums von 1 Stunde vor oder zu Beginn von Schritt ii) zum Reaktionsgemisch zugegeben wird.

5.  Verfahren nach einem beliebigen der voranstehenden Ansprüche, wobei der kurzkettige Alkohol aus einer Gruppe ausgewählt ist, die aus Methanol, Ethanol, Propanol und Kombinationen davon besteht.

6.  Verfahren nach einem beliebigen der voranstehenden Ansprüche, wobei der Wassergehalt des Reaktionsgemisches während der Inkubation im Bereich von 0,3-5 Gew.-% liegt.

7.  Verfahren nach einem beliebigen der voranstehenden Ansprüche, wobei in Schritt ii) das Reaktionsgemisch unter solchen Bedingungen inkubiert wird, dass die Phospholipide des Ölmaterials zu 20% oder mehr zu FAAE umgewandelt werden.

8.  Verfahren nach einem beliebigen der voranstehenden Ansprüche, wobei Phospholipide in dem phospholipidhaltigen Ölmaterial zu FFA und FAAE (FFA:FAAE) in einem Verhältnis umgewandelt werden, das kleiner als oder gleich 80:20 und größer als oder gleich 10:90 ist.

9.  Verfahren nach einem beliebigen der voranstehenden Ansprüche, wobei ein oder mehrere phospholipidabbauende Enzyme eine oder mehrere Phospholipasen umfassen.

10. Verfahren nach einem beliebigen der voranstehenden Ansprüche, wobei das eine oder die mehreren phospholipidabbauenden Enzyme eine Phospholipase A1 aus *Thermomyces lanuginosus* und eine Phospholipase C mit Aktivität gegenüber Phosphatidylinositol umfassen.

11. Verfahren nach einem beliebigen der voranstehenden Ansprüche, wobei die Dosierung von Phospholipase A1 im Bereich von 0,05-1,0 mg Enzymprotein/kg phospholipidhaltiges Öl liegt.

12. Verfahren nach einem beliebigen der voranstehenden Ansprüche, wobei die Menge an Lipaseaktivität im Reaktionsgemisch so ist, dass 5% oder weniger der im Reaktionsgemisch vorhandenen Triglyceride während der Inkubation in Schritt ii) von Anspruch 1 umgesetzt werden.

13. Verfahren nach einem beliebigen der voranstehenden Ansprüche, wobei ein Gemisch aus dem phospholipidhaltigen Ölmaterial und dem kurzkettigen Alkohol mit dem einem oder den mehreren phospholipidabbauenden Enzymen und Wasser vermischt wird.

14. Verfahren nach einem beliebigen der voranstehenden Ansprüche, wobei das Verfahren als eine Vorbehandlung vor einer chemisch katalysierten Umesterung des phospholipidhaltigen Ölmaterials verwendet wird.

**15.** Verfahren nach einem beliebigen der voranstehenden Gegenstände, wobei das umgesetzte Ölmaterial als ein Ausgangsmaterial in einem Umesterungsverfahren verwendet wird oder zur Biodieselproduktion verwendet wird, und wobei vor dem Umesterungsverfahren oder der Biodieselproduktion keine oder im Wesentlichen keine Trennung dieser hydrophoben Phase und dieser hydrophilen Phase erfolgt.

**16.** Verwendung einer Phospholipase zum Katalysieren der Bildung von Fettsäurealkylestern/Biodiesel in einem Reaktionsgemisch, das ein phospholipidhaltiges Ölmaterial, einen kurzkettigen Alkohol mit 1 bis 5 Kohlenstoffatomen und Wasser umfasst, wobei die Menge des Alkohols im Reaktionsgemisch im Bereich von 1-2,5 Gew.-% liegt.

## Revendications

**1.** Procédé de réduction de phospholipides comprenant

i) la fourniture d'un mélange réactionnel comprenant un matériau huileux contenant des phospholipides, une ou plusieurs enzymes dégradant les phospholipides, un alcool à chaîne courte ayant 1 à 5 atomes de carbone, et de l'eau, et
ii) l'incubation du mélange réactionnel ;

dans lequel, lors de l'incubation dudit mélange réactionnel, la quantité dudit alcool à chaîne courte dans le mélange réactionnel est située dans la plage allant de 1 à 2,5 % en poids,
et en outre dans lequel le mélange réactionnel est incubé dans l'étape ii) pour fournir un matériau huileux ayant réagi.

**2.** Procédé selon la revendication 1, dans lequel le matériau huileux ayant réagi comprend une phase hydrophobe comprenant des glycérides ; et une phase hydrophile comprenant de l'eau, un alcool, du phosphore et des enzymes dégradant le phosphore.

**3.** Procédé selon l'une quelconque des revendications précédentes, dans lequel, dans l'étape ii), le mélange réactionnel est incubé dans des conditions permettant auxdites une ou plusieurs enzymes dégradant les phospholipides de catalyser l'hydrolyse et/ou la transestérification de phospholipides dans ledit matériau huileux.

**4.** Procédé selon l'une quelconque des revendications précédentes, dans lequel ladite quantité dudit alcool à chaîne courte ayant 1 à 5 atomes de carbone est ajoutée au mélange réactionnel dans l'heure précédant le ou au début de l'étape ii).

**5.** Procédé selon l'une quelconque des revendications précédentes, dans lequel ledit alcool à chaîne courte est choisi dans le groupe constitué par le méthanol, l'éthanol, le propanol et leurs combinaisons.

**6.** Procédé selon l'une quelconque des revendications précédentes, dans lequel la teneur en eau du mélange réactionnel durant l'incubation est située dans la plage allant de 0,3 à 5 % en poids.

**7.** Procédé selon l'une quelconque des revendications précédentes, dans lequel, dans l'étape ii), le mélange réactionnel est incubé dans des conditions telles que les phospholipides du matériau huileux soient convertis en FAAE à raison de 20 % ou plus.

**8.** Procédé selon l'une quelconque des revendications précédentes, dans lequel les phospholipides dans le matériau huileux contenant des phospholipides sont convertis en FFA et FAAE (FFA/FAAE) en un rapport qui est inférieur ou égal à 80/20 et supérieur ou égal à 10/90.

**9.** Procédé selon l'une quelconque des revendications précédentes, dans lequel une ou plusieurs enzymes dégradant les phospholipides comprennent une ou plusieurs phospholipases.

**10.** Procédé selon l'une quelconque des revendications précédentes, dans lequel lesdites une ou plusieurs enzymes dégradant les phospholipides comprennent une phospholipase A1 de *Thermomyces lanuginosus* et une phospholipase C ayant une activité vis-à-vis du phosphatidylinositol.

**11.** Procédé selon l'une quelconque des revendications précédentes, dans lequel la dose de phospholipase A1 est située dans la plage allant de 0,05 à 1,0 mg de protéine enzymatique par kg d'huile contenant des phospholipides.

12. Procédé selon l'une quelconque des revendications précédentes, dans lequel la quantité d'activité de lipase dans ledit mélange réactionnel est telle que 5 % ou moins des triglycérides présents dans le mélange réactionnel réagissent durant l'incubation dans l'étape ii) de la revendication 1.

13. Procédé selon l'une quelconque des revendications précédentes, dans lequel un mélange dudit matériau huileux contenant des phospholipides et dudit alcool à chaîne courte est mélangé avec une ou plusieurs enzymes dégradant les phospholipides et de l'eau.

14. Procédé selon l'une quelconque des revendications précédentes, ledit procédé étant utilisé en tant que prétraitement avant transestérification chimiquement catalysée dudit matériau huileux contenant des phospholipides.

15. Procédé selon l'un quelconque des revendications précédentes, dans lequel ledit matériau huileux ayant réagi est utilisé comme matière première dans un procédé de transestérification ou est utilisé pour la production de biodiésel, et dans lequel il n'y a pas ou pratiquement pas de séparation de ladite phase hydrophobe et de ladite phase hydrophile avant le procédé de transestérification ou la production de biodiésel.

16. Utilisation d'une phospholipase pour catalyser la formation d'esters alkyliques d'acides gras / de biodiésel dans un mélange réactionnel comprenant un matériau huileux contenant un phospholipide, un alcool à chaîne courte ayant 1 à 5 atomes de carbone, et de l'eau, dans laquelle la quantité dudit alcool dans le mélange réactionnel est située dans la plage allant de 1 à 2,5 % en poids.

# Figure 1

# Figure 2

Figure 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5264367 A
- JP 2153997 A
- EP 622446 A
- WO 2016064848 A
- EP 1893764 A
- WO 2003100044 A
- WO 2004064537 A
- WO 2005066347 A
- WO 2008019069 A
- WO 2009002480 A
- WO 2009081094 A
- WO 2008036863 A
- WO 200032758 A

**Non-patent literature cited in the description**

- **BOCHISCH, M.** Fats and Oils Handbook. AOCS Press, 1998, 428-433
- **CLAUSEN, K**. *Eur. J. Lipid Sci. Techno.*, 2001, vol. 103, 333-340
- **CLAUSEN et al.** *Dansk kemi*, 2002, vol. 88, 24-27
- **CESARINI et al.** *Biotechnology for Biofuels*, 2014, vol. 7, 29
- **CESARINI et al.** *Sustainability*, 2015, vol. 7, 7884-7903
- **LI et al.** *Biotechnology and Bioprocess Engineering*, 2015, vol. 20, 965-970
- **NIELSEN et al.** *Protein Engineering*, 1997, vol. 10, 1-6
- **NEEDLEMAN ; WUNSCH**. *J. Mol. Biol.*, 1970, vol. 48, 443-453
- **RICE et al.** EMBOSS: The European Molecular Biology Open Software Suite. *Trends Genet*, 2000, vol. 16, 276-277